# EUROPEAN PATENT SPECIFICATION

(11) **EP 3 247 435 B1**
(45) Date of publication and mention of the grant of the patent: **10.03.2021**
(21) Application number: 16740889.7
(22) Date of filing: 23.01.2016
(51) Int. Cl.: A61M 15/00, B05B 17/06, A61M 15/06, B06B 1/06

(54) **ULTRASONIC VAPORIZING ELEMENT**
ULTRASCHALL-VERDAMPFUNGSELEMENT
ÉLÉMENT DE VAPORISATION PAR ULTRASONS

(30) Priority: 23.01.2015 US 201562106852 P; 02.04.2015 US 201562142464 P
(43) Date of publication of application: 29.11.2017
(73) Proprietor: Tan, William, San Gabriel, CA 91776 (US)
(72) Inventor: Tan, William, San Gabriel, CA 91776 (US)
(74) Representative: Schaumburg und Partner Patentanwälte mbB
(86) International application number: PCT/US2016/014646
(87) International publication number: WO 2016/118941

(56) References cited:
- WO-A1-2008/015394
- GB-A- 2 107 611
- JP-A- 2000 051 755
- JP-A- 2010 142 737
- US-A- 4 085 893
- US-A- 5 950 619
- US-A1- 2009 065 600
- US-A1- 2010 044 460
- US-A1- 2010 044 460
- US-B1- 6 278 218
- US-B1- 7 975 688
- US-B2- 7 784 712

## Description

### BACKGROUND OF THE INVENTION

### 1. Field of the Invention:

An improvement for an ultrasonic vaporizing element for an electronic cigarette.

### 2. Description of Related Art

In recent years, portable electronic vaporizers, or commonly known as electronic cigarettes or "e-cigs" have gained popularity among users who vaporize herbal extracts to inhale the vapors emitted when the extracts are heated. This action is often referred to as "vaping." An electronic vaporizer uses one or more batteries to power a heating element, which then heats up a small amount of the vaping liquid at an appropriate temperature to convert the liquid to vapor, which the user then inhales. Vaping liquids are typically solutions of propylene glycol, glycerol, or both, plus nicotine and flavorant chemicals. Medical marijuana users also add cannabis extracts to the mixture. Some E-cigarettes are also designed to allow for vaporization of solid herbal extracts. Various types of liquid and solid herbal extracts are available and are commonly referred to as "e-juice," "essential oil," "butter," "concentrate" or "wax."

A common heating element is Nichrome resistance wire, which is a non-magnetic alloy of nickel, chromium, and often iron. A typical device will contain a rechargeable battery (usually lithium-ion), which is connected to a circuit board that has an On/Off switch. When the switch is turned on, the current from the battery will flow to the heating element to generate heat and causes the herbal extract that is placed on or close to the heating element to vaporize. The user will then inhale the vapor via a mouthpiece. A drawback of using Nichrome as a heat source is that nickel alloys and compounds are classified as carcinogenic to humans:
www.nipera.org/WorkplaceGuide/WorkplaceSurveillance/CarcinogenicClassifications.aspx.

Another disadvantage of the heating system of such vaporizers is that after several uses,
the heating element will be covered with residue from the extract. Over time, the residue will build up and causes a drop in the heater's efficiency. At such point, the device will be rendered inoperable, and the user will need to replace or repair the heating element.

Health issues associated with "vaping" have also been reported by The New England Journal of Medicine (www.neim.org/doi/full/10.1056/NEJMc1413069). At heated temperatures, formaldehyde, a known carcinogen, is released.

Ultrasonic atomization: a more effective method for vaporizing herbal extracts is thus explored via ultrasonic atomization. Ultrasonic atomization or nebulization has been studied for decades and much has been written about these subjects and shall not be elaborated in detail here. Briefly, atomization occurs through the rapid mechanical upward and downward motion of an ultrasonic tip, which causes a film of liquid to form into standing capillary waves.

When the amplitude of the capillary wave peaks above what is required for stability of the system, the liquid at the peak crests breaks away in the form of droplets. The phenomenon known as cavitation occurs at higher energy levels. Microscopic gas bubbles in a liquid will be forced to oscillate due to the applied sound wave. At such high intensity, bubbles will grow in size and rapidly collapse or implode. This disintegration of the liquid also results in droplet formation. Ultrasonic atomization is often used in automotive spray painting, in humidifiers, and coatings for fuel cells, wafers and solar panels.

Ultrasonic sound waves are generally created by piezoelectric or magnetostrictive transducers. Piezoelectric transducers utilize the piezoelectric property of a material to convert electrical energy directly into mechanical energy. Magnetostrictive transducers utilize the magnetostrictive property of a material to convert the energy in a magnetic field into mechanical energy.

Investigation into the suitable ultrasonic frequency and amplitude is made using an Ultrasonic Processor, such as the Hielscher UP100H model; this is lab equipment used for sonochemistry, which is the study of emulsifying, dispersing, dissolving and cell disruption of liquids. A frequency range of 20 - 40 kHz and a displacement amplitude of at least 10 micrometer at the vibrating tip is observed to sufficiently atomize a thin layer of vaping liquid with comparable vapor volume as that generated by traditional electronic cigarettes.

However, using a lab ultrasonic processor will be impractical as a portable vaporizer due to the size, weight and high power requirement. Another important consideration is the method of containing and delivering the vaping liquid to the vibrating ultrasonic tip (sonotrode or horn). Using a separate beaker like that used in conjunction with a lab ultrasonic processor will not be leak proof or practical.

Nebulizers: other types of vaporizers in the medical industry exist whereby liquids are atomized using ultrasonic sound waves. These types of vaporizers are also called nebulizers, which is a drug delivery device used to administer medication in the form of a mist inhaled into the lungs to treat cystic fibrosis, asthma, COPD and other respiratory diseases. Different types of ultrasonic nebulizers are described below:
Ultrasonic wave nebulizers - these have an electronic oscillator generate a high frequency ultrasonic wave, which causes the mechanical vibration of a Mist Transducer. This transducer is comprised of a ring-shaped piezoelectric element attached to a metal plate, which amplifies the vibration. The metal plate is in contact with a liquid reservoir, and its high frequency vibration is sufficient to produce a vapor mist. An example of such a type of Mist Transducer is the SMIST15F28RR111 model produced by Steiner and Martins, Inc. of Florida, USA. Examples of such nebulizers are: Omron NE-U17 and Beurer Nebulizer IH30. These nebulizers are often larger table top devices requiring plug-in power.

Vibrating mesh technology (VMT): a metallic mesh/membrane with 1000-7000 laser drilled holes vibrates at the top of the liquid reservoir, and thereby pressures out a mist of very fine droplets through the holes. This technology is more efficient than having a vibrating piezoelectric element at the bottom of the liquid reservoir, and thereby allows for smaller and more portable designs. The mesh can be vibrated with the same ring-shaped piezoelectric element attached to the mesh, or it can be vibrated by a Langevin type transducer placed against the mesh; some examples are Pari eFlow, Respironics i-Neb, Beurer Nebulizer IH50, Aerogen Aeroneb and Omron MicroAir products.

Sandwich type ultrasonic transducers, also called bolt-clamped or Langevin transducers, are well known and established for the production of high intensity ultrasonic motion. In United Kingdom Patent No. 145,691, issued in 1921, P. Langevin inventor, a sandwich of piezoelectric material positioned between metal plates is described to generate high intensity ultrasound. Sandwich transducers utilizing a bolted stack transducer tuned to a resonant frequency and designed to a half wavelength of the resonant frequency are described in United Kingdom Patent No. 868,784.

Regardless of the types of nebulizer technology, these devices are inappropriate for vaping liquids or solids used in electronic cigarettes. The drugs used for nebulizing are often water-like in viscosity (water = 1cSt), whereas vaping liquids are often more than 50cSt, depending on the types and concentration of glycol, glycerol and nicotine in the mixture. Bubbling is observed when vaping liquids are used in medical nebulizers, but the intensity of the vibration is insufficient to cause atomization.

An obvious solution to the aforementioned problem is to use a higher-powered transducer. However, this is unsuitable for the following reasons:
1. Size - The ring-shaped transducers used in ultrasonic wave or VMT nebulizers are often 1.5 cm to 4 cm in diameter. For such transducers to be powerful enough to atomize viscous vaping liquids, the transducer has to be significantly bigger for higher amplification. This is apparently unsuitable to be incorporated in a pocket-sized vaporizer.
2. Power - A higher power input is required for powering a larger transducer, which will be unsuitable for a portable device running on battery.
3. Noise - A VMT nebulizer utilizing a Langevin transducer will generate a very noticeable high-pitch noise if the transducer sonotrode or tip vibrates at the desired amplitude of more than 10 micrometer against metal or any hard objects. This rules out any transducer-to-metal mesh vibration mechanism.

Another consideration is the cleanability of the device. Vaping liquids containing cannabis extracts, or solid cannabis extracts are thick, sticky and do not dissolve in water or detergent. At room temperature, these liquids and are difficult to wash off without acidic solvents. Thus, any liquid tank in the portable device should preferably be replaceable and disposable.

However, existing nebulizers using VMT often incorporate the mesh and transducer directly onto the removable liquid tanks and are not intended to be discarded after short uses. The preferred configuration should be to separate the tank from the transducer or vibrating source so that the tank itself can be cheaply replaced. This presents a challenge with the appropriate

methodology of delivering the vaping liquid from a removable tank to the transducer that is permanently attached to the device, and yet is leak proof when transported. From the United States patent application publication US 2010/0044460 A1 an ultrasound liquid atomizer comprising a rigid piezoelectric transducer body is known. From the United Kingdom patent GB 2 107 611 A a liquid sprayer suitable for spraying water particles into the nose or mouth of a human or animal is known. From the Japanese patent application publication JP 2000 051755 A a vibrating mesh technology (VMT) nebulizer having a liquid absorbent core is known. Further, from the Japanese patent application publication JP 2010 142737 A a nebulizer having the structural features as described in the precharacterizing part of claim 1 is known.

It is an object to improve an apparatus for ultrasonic atomizing of a vaping liquid with respect to better cleanability of the apparatus including improved detachability and leak proof transportability of a vaping liquid tank.

This and other objects are achieved by the current invention as defined in claim 1. . Advantageous further embodiments are claimed in the dependent claims.

### BRIEF DESCRIPTION OF THE DRAWINGS

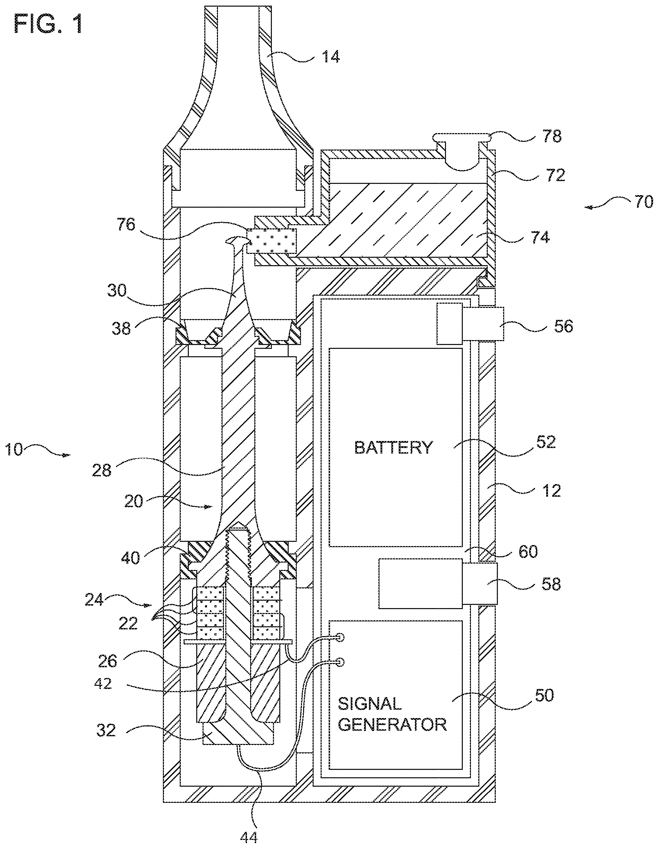

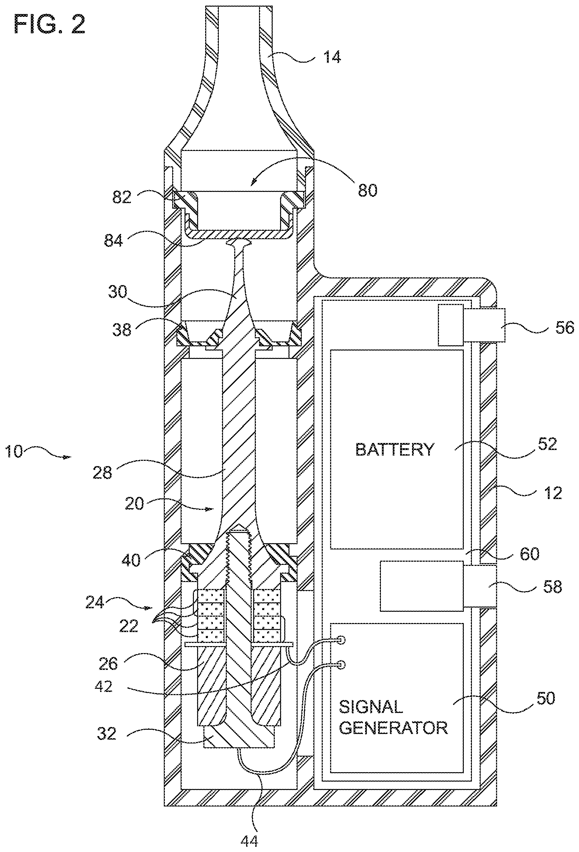

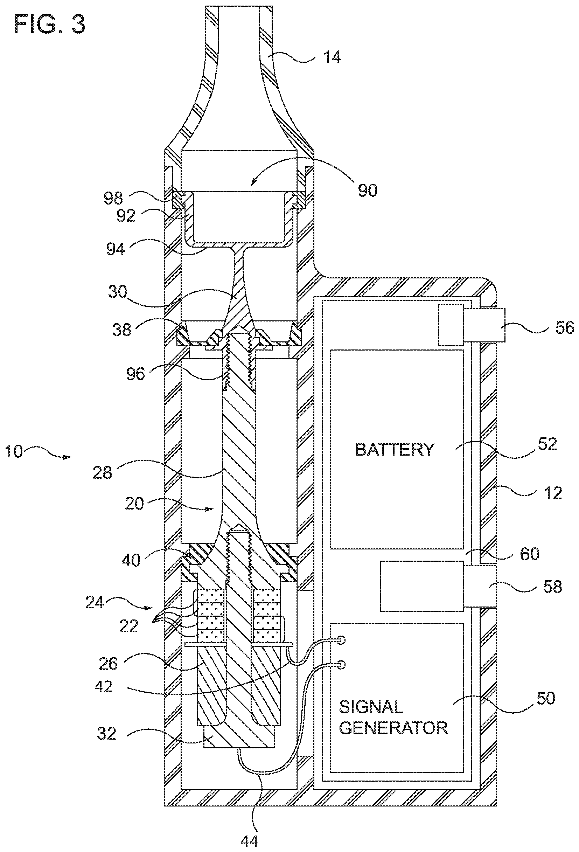

FIG. 1 is a longitudinal cross-sectional view of an embodiment of the nebulizer with a removable liquid tank according to the invention. FIG. 2 is a longitudinal cross-sectional view of a comparative example of nebulizer with a removable soft membrane container. FIG. 3 is a longitudinal cross-sectional view of a further comparative example of nebulizer with a removable solid tank container incorporated into the sonotrode.

### Parts Listing:

10 nebulizer
12 body
14 mouthpiece
20 ultrasonic transducer assembly or transducer assembly
22 piezoelectric elements
24 transduction portion
26 "anvil" or bearing member
28 amplification member or "sonotrode"
30 velocity transformer
32 bolt
38, 40 acoustic isolators
42, 44 electric current supply wires
50 high frequency signal generator
52 battery or power source
56 activation actuator or on/off switch
58 input power jack
60 circuit board
70 tank assembly
72 tank
74 vaping liquid or substance
76 soft absorbent material or "wick"
78 stopper
80 removable container
82 container side wall
84 soft bottom membrane
90 container
92 side wall of container
94 container bottom

### DESCRIPTION OF THE PREFERRED EMBODIMENTS

Referring to Figures 1-3, there is illustrated several examples for the vaporizing element for a certain type of e-cigarette or "e-cig," - each said example employs an ultrasonic signal generator to vaporize the e-juice (or material to be vaporized, including without limitation "wax," gel or solid vaporizable substances) from the e-juice container or tank.

This invention uses and employs ultrasonic signals and vibrations to cause atomization of e-juice, such as a nebulizer. There are existing nebulizers, such as: (http://www.omron-healthcare.com/eu/en/our-products/respiratory-therapy/microair-u22), but these nebulizers work with thin liquids that are water-like (viscosity = 1 Cst), and are not powerful enough for viscous liquids >10 Cst.

The inventor has discovered such a suitable frequency is about 30 kHz, and the amplitude of the vibrating probe in contact with liquid at >10 micrometer. A higher displacement will create more vapor output. Lower frequencies can also atomize, but the vapor particles will be too large. It is presumed that no prior art vaporizers or nebulizers exist that work in the desired frequency/amplitude range. Ultrasonic lab equipment for sonochemistry exists for cavitation of liquids and is powerful enough for atomizing e-juice.

However, these existing lab equipment devices are high-powered and often have very long ultrasonic "horns" or probes that amplify the signal. This setup makes it ineffective for a portable device.

The discovery of shaping a transducer horn so that amplitude can be increased has been important; it is L-shaped (like dental scaling tips). This takes up less space with similar vibrational displacement achieved at the end of the probe. Due to the vibration energy created, especially at the end of the probe, heat is generated. This can reach >70°C, which means that the device can also be used for solids or waxes such as THC. THC melts at 66°C and turns into a liquid, which can then be ultrasonically atomized.

A preferred embodiment of an ultrasonic atomizing/nebulizing device, which generally comprises: an ultrasonic generator (typical, existing), powered by a DC power supply (can be battery or DC from an AC/DC converter); an ultrasonic generator drives a Langevin transducer; a horn, a part of the transducer, is a typical component of a Langevin transducer to amplify the ultrasonic signal, but not sufficient to atomize viscous liquid; a metal probe is attached to horn; a probe is bent to allow for greater movement of the probe tip so as to create a displacement of >10 micrometer; a tip touches a cotton wick, which draws fluid from a removable tank; and vapor of the atomized substance or liquid exits the mouthpiece.

In Figures 1 to 3, the nebulizer **10** comprises: a body **12,** comprising a top portion mouthpiece **14,** and encloses an ultrasonic transducer assembly **20** constituted by a stack of piezoelectric elements **22** which are supplied with electric current by supply wires **42** and **44** connected to a high frequency signal generator **50,** which is an integral component on circuit board **60** which also includes primarily an on/off switch **56,** a battery power source **52,** and an input power jack **58.** The transducer assembly **20** is held within the nebulizer housing **12** with acoustic isolators **40** and **38,** so as to dampen the vibrations emitted from the transducer assembly **20** to the housing **12.** Acoustic isolator **38** also acts as a seal to prevent nebulized droplets from straying into other components of the nebulizer **10.**

The transducer assembly **20,** which is known as a "Langevin stack," generally includes a transduction portion **24,** a bearing member or "anvil" **26,** and an amplification member or "sonotrode" **28.** The bearing member or "anvil" **26** is connected to the proximal end of transduction section **24,** and the sonotrode **28** is connected to the distal end of transduction portion **24.** The anvil **26,** sonotrode **28** are preferably fabricated from titanium, aluminum, stainless steel, or any other suitable material. Sonotrode **28** and anvil **26** have a length determined by a number of variables, including the thickness of the transduction section **24,** the density and modulus of elasticity of material used in sonotrode **28,** anvil **26** and the resonant frequency of the transducer assembly **20.** The sonotrode **28** may be tapered inwardly from its proximal end to its distal end to amplify the ultrasonic vibration amplitude as velocity transformer **30,** or alternately may have no amplification.

The piezoelectric elements **22** may be fabricated from any suitable material, such as, for example, lead zirconate-titanate, lead meta-niobate, lead titanate, or other piezoelectric crystal material. The piezoelectric elements **22** have a bore extending through the center and are electrically coupled to wires **42** and **44,** and electrically connected to the signal generator **50** on circuit board **60.** The circuit board **60** also comprises a power source in the form of a rechargeable battery **52,** an on/off switch **56** and a power jack **58** for accepting an external power source for charging the rechargeable battery **52** or to power the circuit board **50** in the absence of battery **52.** Alternatively, the power jack **58** can be omitted if the battery **52** is removable from the nebulizer **10** for charging externally, or the nebulizer **10** is powered only by plug-in power through the power jack **58** without a battery power source.

The piezoelectric elements **22** are conventionally held in compression between anvil **26** and sonotrode **28** by a bolt **32.** The bolt **32** preferably has a head, a shank, and a threaded distal end. The bolt **32** is inserted from the proximal end of anvil **26** through the bores of anvil **26** and piezoelectric elements **22.** The threaded distal end of the bolt **32** is screwed into a threaded bore in the proximal end of sonotrode **28.** A removable mouthpiece **14** is attached to the nebulizer housing **12** to direct the vapor generated for inhalation by the user.

In order for the transducer assembly **20** to deliver energy all components of transducer assembly **20** must be acoustically coupled. The components of the transducer assembly **20** are preferably acoustically tuned such that the length of any assembly is an integral number of one-half wavelengths (nλ/2), where the wavelength λ is the wavelength of a pre-selected or operating longitudinal vibration drive frequency f_{d} of the acoustic assembly **20,** and where n is any positive integer. It is also contemplated that the acoustic assembly **20** may incorporate any suitable arrangement of acoustic elements.

Referring to FIGS. 1 to 3, wires **42** and **44** transmit the electrical signal from the signal generator **50** to the piezoelectric elements **22** of the transducer assembly **20.** The signal generator **50** is in turn electrically powered by a battery **52,** and drives the circuit board **60** that primarily includes the signal generator **50,** on/off switch **56** and power input jack **58.** The piezoelectric elements **22** are energized by an electrical signal supplied from the generator **50** in response to the on/off switch **56** to produce an acoustic standing wave in the transducer **20.** The electrical signal causes disturbances in the piezoelectric elements **22** in the form of repeated small displacements resulting in large compression forces within the material. The repeated small displacements cause the piezoelectric elements **22** to expand and contract in a continuous manner along the axis of the voltage gradient, producing longitudinal waves of ultrasonic energy. The ultrasonic energy is transmitted through the acoustic assembly **20** to the velocity transformer **30.** Vaping medium or substance in contact with the distal end of the velocity transformer **30** is energized or agitated to the point of atomization or cavitation, resulting in nebulization of the vaping medium.

In FIG. 1 of the embodiment of the present invention, a removable tank assembly **70** is attached to the housing **12** to store an amount of vaping liquid **74** within the tank **72.** On one opening end of the tank **72,** a soft absorbent material or "wick" **76** is adapted to draw a small amount of vaping liquid **74** to the distal end of the velocity transformer **30.** The wick **76** is made from materials, including without limitation: cotton, fiberglass, ceramic fiber or any material that is absorbent and acoustically dampening, so that any friction caused by contact with the distal end of the velocity transformer **30** does not result in unnecessary friction-induced high pitched noise. The wick **76** is placed appropriately so that there is sufficient contact with the distal end of the velocity transformer **30,** and allows for a capillary amount of vaping liquid to flow onto the distal end of the velocity transformer **30** for nebulization. As more of the liquid is nebulized, the wick **76** continues to draw more liquid **74** from the tank **72** to the velocity transformer **30.** When the tank assembly **70** is removed from the nebulizer housing **12,** the wick **76** acts as a stopper to prevent leakage of the vaping liquid **74.** On another opening end of the tank **72,** a stopper **78** plugs the opening and can be removed to allow refilling of the vaping liquid **74.** The tank assembly **70** can thus be easily removed for cleaning or replaced inexpensively without affecting any components of the transducer assembly **20.**

In FIG. 2 a removable container **80** is adapted between the mouthpiece **14** and nebulizer housing **12,** and in contact with the distal end of the velocity transformer **30.** The container **80** is constructed of a soft bottom membrane **84,** and rigid side-wall **82.** The soft bottom membrane **84** is made from resilient and pliable materials such as silicone or thermoplastic elastomer or any material that can withstand the vibrations of the velocity transformer **30** without melting or breaking, and at the same time acoustically dampening to prevent generating friction-induced noise when the transducer **20** is activated. Further, the thickness of the soft bottom membrane **84** should preferably be thin, about 0.5mm (0.02") so that vibratory energy can be transmitted from the velocity transformer **30** through the material of the bottom piece **84.** The side-wall **82** is made from materials such as plastic, metal, glass or ceramic. The soft bottom **84** can be over-molded onto the side-wall **82,** or it can be stretched over the side-wall **82** as a two-piece constructed tank **80.**

Whichever method the tank **80** is constructed, the soft bottom membrane **84** should preferably be taut around the side-wall **82,** so that when the bottom **80** is in contact with the velocity transformer **30,** vibratory energy can be efficiently transferred to the content of container **80.** Vaping liquid or solid placed inside the container **80** can thus be energized by the velocity transformer **30** for nebulization without being in direct contact with the velocity transformer **30.** Such vaping liquid or solid used in this example are typically gel-like or wax-like with high viscosity and do not flow freely through a wick **76** as described in Fig. 1. The container **80** described in this example can thus be easily removed for cleaning or replaced inexpensively without affecting any components of the transducer assembly **20.**

In FIG. 3 a solid container **90** is integrated as part of the velocity transformer **30.** The velocity transformer **30** has a first end and a second end. The sonotrode **28** comprises a first end coupled to the transduction section **24** of the transducer **20.** The second end of the sonotrode **28** has a threaded distal end coupled to the first end of the velocity transformer **30.** The second end of the velocity transformer has an attached container **90,** comprising a bottom **94** and side-wall **92.** An acoustic isolator **98** is adapted between the container **90** and nebulizer housing **12** to minimize any transfer of vibration and friction to the housing **12.** The velocity transformer **30** and attached container **90** can thus be removed from the sonotrode **28** by unscrewing from the sonotrode **28** for cleaning or storage. When the velocity transformer **30** is coupled to the sonotrode **28,** ultrasonic energy from the acoustic assembly **20** is transferred to the bottom **94** of the container **90.** Vaping liquids or solids placed inside the container **90** can thus be energized by the velocity transformer **30** for nebulization. Such vaping liquid or solid used in this example are typically gel-like or wax-like with high viscosity and do not flow freely through a wick **76** as described in FIG. 1.

The container **90** and the attached velocity transformer **30** can be made from a single metallic material such as titanium, stainless steel or aluminum. Alternatively, the container **90** and velocity transformer **30** are separate components attached together with glue or fasteners. In either case, the container **90** and velocity transformer **30** should be attached in such a manner as to allow the maximum transfer of ultrasonic energy from the acoustic assembly **20** to the bottom **94** of the tank **90,** without looseness or allowing for friction between the container **90** and velocity transformer **94.** Any looseness or friction will result in audible and undesired high-pitched friction noise. Transducers are currently available and are used in several devices like dental scalers and surgical knives, and existing devices are shown and described in the following patents: US 6278218; US 5702360; and US 8257377.

There are 3 examples of design. The first one is object of the current invention and is for liquid and are the most applicable for e-cigarettes. The other two are more suitable for concentrates and THC waxes.

Embodiment 1 for liquids: For the embodiment of the current invention, the most important component is the delivery of the liquid from the tank to the transducer using a wick. No ultrasonic nebulizers for medicine use a wick; most of them have transducers, which are attached directly to a container to fill the liquid. Using a wick limits the amount of liquid to the transducer, such that it is just enough for atomization. Using a container like existing nebulizers requires the transducer has to be so powerful as to transfer energy through the depth of the liquid, but with instant improved design, less power is required.

Example 1: for the first example, using silicone to atomize THC is a big discovery because the energy from the transducer can pass through silicone to melt and then atomize the THC, but it is important to have the silicone material taut.

Note that hard materials will not work, such as metal, glass or ceramic; the transducer scratches the material and creates an audible screeching sound. If in plastic, the transducer actually melts and cut through the plastic, much like an ultrasonic welding machine. Silicone has another advantage because THC material does not stick to the silicone and makes for easier cleaning.

Example 2: the second example improves on Example 1 (silicone) and has a metal container that is attached to the transducer so there is as little as possible friction between the two parts; little or no friction results in reduced or no sound.

The function of a typical nebulizer is directly related to the amount of liquid above the probe (or velocity transformer) of the transducer. The thicker the amount of liquid above the probe, the more powerful the transducer needs to be in order to transmit the acoustic energy to the surface of the liquid to cause atomization.

To minimize the amount of power required, a thin film of liquid is desired, so that less energy (thus smaller battery) is sufficient to atomize the liquid; the preferred wick will draw just enough liquid to the probe so that no big puddle is formed on the probe (i.e., big puddle = thick amount = insufficient energy to transmit through the thickness of the liquid).

Conversely, if the wick is too dense, not enough liquid is drawn to the probe for optimal vapor output. The liquid flow amount of the wick can be controlled by the wick's density, porosity, and/or constricting the wick's diameter.

Detailed embodiments of the present invention are disclosed; however, the disclosed embodiments are merely exemplary of the invention, which can be embodied in various forms; specific structural and functional details disclosed are not to be interpreted as limiting, but merely as a basis for the claims and as a representative basis for teaching one skilled in the art to variously employ the present invention in virtually any appropriately detailed structure. The title, headings, terms and phrases used are not intended to limit the subject matter or scope; but rather, to provide an understandable description of the invention. The invention is composed of several sub-parts that serve a portion of the total functionality of the invention independently and contribute to system level functionality when combined with other parts of the invention. The terms "a" or "an" are defined as: one or more than one. The term "plurality" is defined as: two or more than two. The term "another" is defined as: at least a second or more. The terms "including" and/or "having" are defined as comprising (i.e., open language). The term "coupled" is defined as connected, although not necessarily directly, and not necessarily mechanically.

## Claims

1. An apparatus (10) for ultrasonic atomizing of a liquid (74) in a replaceable container (72), comprising:
a housing (12) with a power source (52);
wherein the housing (12) has the container (72) with the liquid (74) for atomizing;
an ultrasonic transducer (24) and a sonotrode (28), wherein the container (72) has a wick (76) for drawing the liquid (74) to the sonotrode (28) provided at one end of the ultrasonic transducer (24), which is located in the housing (12);
the sonotrode (28) being configured to engage the wick (76) and allow for a displacement of greater than 10 micrometer;
the ultrasonic transducer (24) is connected to the power source (52) and a signal generator (50);
the ultrasonic transducer (24) is a Langevin stack with piezoelectric elements (22) held in compression between an anvil (26) and the sonotrode (28) by a bolt (32), transmitting ultrasonic energy to the sonotrode's forward end that engages the wick formed by a porous absorbent material interfacing the forward end of the sonotrode, wherein the liquid in the replaceable container is such that the liquid is delivered to the sonotrode for nebulizing in a controlled manner;
whereby when activated, the ultrasonic transducer (24) is configured to vibrate the sonotrode (28), such that the liquid (74) from the wick (76) is atomized, and vapor of the atomized liquid exits the apparatus; and
a removable container assembly (70) that is removable from the housing (12);
**characterized in that**
the housing (12) has a mouthpiece (14) and the liquid is a vaping liquid; and
the wick (76) is configured to act as a stopper to prevent leakage of the vaping liquid (74) when the container assembly (70) is removed from the nebulizer housing (12).

2. The apparatus of claim 1 wherein the power source (52) is an internal battery or an external power source; and the ultrasonic transducer (24) is activated by an activation switch (56) or an activation button (56).

3. The apparatus of claim 1 wherein the housing (12) has acoustic isolators (38, 40) to dampen vibrations emitted from the ultrasonic transducer (24) to the housing (12).

## Patentansprüche

1. Eine Vorrichtung (10) zum Vernebeln einer in einem austauschbaren Behälter (72) befindlichen Flüssigkeit (74) durch Ultraschall, mit:
einem Gehäuse (12) mit einer Stromquelle (52);
wobei das Gehäuse (12) zum Vernebeln den mit der Flüssigkeit (74) befüllten Behälter (72) enthält;
einem Ultraschallschwinger (24) und einer Sonotrode (28), wobei der Behälter (72) einen Docht (76) aufweist, welcher die Flüssigkeit (74) zu der Sonotrode (28) hin saugt, welche an einem Ende des Ultraschallschwingers (24) angeordnet ist, welcher sich in dem Gehäuse (12) befindet;
wobei die Sonotrode (28) derart ausgebildet ist, dass diese mit dem Docht (76) in Eingriff bringbar ist, um eine Auslenkung von mehr als 10 µm zu ermöglichen;
wobei der Ultraschallschwinger (24) mit der Stromquelle (52) und einem Signalgeber (50) verbunden ist;
der Ultraschallschwinger (24) ein Langevin-Stapel mit piezoelektrischen Elementen (22) ist, welche von einem Bolzen (32) zwischen einem Gegenhalter (26) und der Sonotrode (28) unter Druck gehalten sind, wobei die Ultraschallenergie zu dem vorderen Ende der Sonotrode geleitet wird, welches sich mit dem Docht im Eingriff befindet, welcher aus einem porösen, absorbierenden Material ausgebildet ist, welches dem vorderen Ende der Sonotrode zugewandt ist, wobei die sich in dem austauschbaren Behälter befindliche Flüssigkeit zum gesteuerten Vernebeln zu der Sonotrode hin gefördert wird;
wobei der Ultraschallschwinger (24), wenn aktiviert, die Sonotrode (28) vibrieren lässt, so das die Flüssigkeit (74) von dem Docht (76) vernebelt wird, so dass die vernebelte Flüssigkeit die Vorrichtung verlässt; und
einer austauschbaren Behälteranordnung (70), welche von dem Gehäuse (12) entfernbar ist;
**dadurch gekennzeichnet, dass**
das Gehäuse (12) ein Mundstück (14) aufweist und die Flüssigkeit eine Vape-E-Flüssigkeit ist; und
der Docht (76) derart ausgebildet ist, dass dieser als ein Verschlussstopfen dient, welcher eine Leckage der Vape-E-Flüssigkeit (74) verhindert, während die austauschbare Behälteranordnung (70) von dem Vernebler-Gehäuse (12) entfernt ist.

2. Die Vorrichtung nach Anspruch 1, wobei die Stromquelle (52) eine interne Batterie oder eine externe Stromquelle ist; und der Ultraschallschwinger (24) durch Betätigen eines Schalters (56) oder durch Betätigen eines Schaltknopfs (56) aktiviert wird.

3. Die Vorrichtung nach Anspruch 1, wobei das Gehäuse (12) Schalldämmelemente (38,40) aufweist, um die von dem Ultraschallschwinger (24) auf das Gehäuse (12) übertragenen Schwingungen zu dämpfen.

## Revendications

1. Appareil (10) d'atomisation par ultrasons d'un liquide (74) dans un contenant remplaçable (72), comprenant :
un boîtier (12) avec une source d'alimentation (52) ;
dans lequel le boîtier (12) comporte le contenant (72) avec le liquide (74) d'atomisation ;
un transducteur ultrasonore (24) et une sonotrode (28), dans lequel le contenant (72) a une mèche (76) pour attirer le liquide (74) vers la sonotrode (28) prévue à une première extrémité du transducteur ultrasonore (24), qui est située dans le boîtier (12) ;
la sonotrode (28) étant configurée pour être en prise avec la mèche (76) et permettre un déplacement supérieur à 10 micromètres ;
le transducteur ultrasonore (24) est connecté à la source d'alimentation (52) et à un générateur de signaux (50) ;
le transducteur ultrasonore (24) est un empilement de Langevin avec des éléments piézoélectriques (22) maintenus en compression entre une butée (26) et la sonotrode (28) par un boulon (32), transmettant une énergie ultrasonore à l'extrémité avant de la sonotrode qui est en prise avec la mèche formée par un matériau absorbant poreux en interface avec l'extrémité avant de la sonotrode, dans lequel le liquide dans le contenant remplaçable est tel que le liquide est délivré à la sonotrode pour nébulisation d'une manière commandée ;
de telle sorte que lorsqu'il est activé, le transducteur ultrasonore (24) est configuré pour faire vibrer la sonotrode (28) de telle manière que le liquide (74) provenant de la mèche (76) soit atomisé, et que la vapeur du liquide atomisé sort de l'appareil ; et
un ensemble de contenant amovible (70) qui peut être retiré du boîtier (12) ;
**caractérisé en ce que**
le boîtier (12) a un embout buccal (14) et le liquide est un liquide de vapotage ; et
la mèche (76) est configurée pour agir comme un bouchon afin d'empêcher une fuite du liquide de vapotage (74) lorsque l'ensemble de contenant (70) est retiré du boîtier de nébuliseur (12).

2. Appareil selon la revendication 1, dans lequel la source d'alimentation (52) est une batterie interne ou une source d'alimentation externe ; et le transducteur ultrasonore (24) est activé par un commutateur d'activation (56) ou un bouton d'activation (56).

3. Appareil selon la revendication 1, dans lequel le boîtier (12) a des isolants acoustiques (38, 40) pour amortir les vibrations émises par le transducteur ultrasonore (24) vers le boîtier (12).
